# EUROPÄISCHE PATENTANMELDUNG

(11) **EP 4 588 441 A1**
(43) Veröffentlichungstag der Anmeldung: **23.07.2025**
(21) Anmeldenummer: 24152388.5
(22) Anmeldetag: 17.01.2024
(51) Int. Cl.: A61B 6/00

(54) **VERFAHREN ZUM BETRIEB EINER RÖNTGENEINRICHTUNG, RÖNTGENEINRICHTUNG, COMPUTERPROGRAMM UND ELEKTRONISCH LESBARER DATENTRÄGER**

(71) Anmelder: Siemens Healthineers AG, 91301 Forchheim (DE)
(72) Erfinder: Fabricius, Uwe, 91056 Erlangen (DE); Kowarschik, Markus, 91052 Erlangen (DE); Pfister, Marcus, 91088 Bubenreuth (DE); Roser, Philipp, 91052 Erlangen (DE)
(74) Vertreter: Siemens Healthineers Patent Attorneys

(57) **Zusammenfassung**

Die Erfindung betrifft ein Verfahren zum Betrieb einer Röntgeneinrichtung (6), welche aufweist:
- eine Röntgenstrahleranordnung (9),
- einen Röntgendetektor (10) zum Empfang von Röntgenstrahlung eines von der Röntgenstrahleranordnung (9) ausgesendeten Röntgenstrahlenfeldes (13),
- eine Bilderzeugungskette (15) zur Ermittlung eines Röntgenbildes (1, 4) eines Aufnahmebereichs (14) aus mit dem Röntgendetektor (10) aufgenommenen Rohdaten, und
- eine Ausgabeeinrichtung (17) zur Ausgabe eines das Röntgenbild (1, 4) umfassenden oder daraus ermittelten Ausgabebildes (5),

wobei das Verfahren folgende Schritte umfasst:
- Aufnahme einer Abfolge von Röntgenbildern (1, 4) eines Aufnahmebereichs (14), insbesondere zur Überwachung des Aufnahmebereichs (14), und
- Ermittlung einer Abfolge auszugebender Ausgabebilder (5) aus den Röntgenbildern (1, 4),

wobei bei der Aufnahme der Röntgenbilder (1, 4) wiederholt zwischen wenigstens zwei Aufnahmeparametersätzen, die zur unterschiedlichen Darstellung wenigstens zweier unterschiedlicher Objekte des Aufnahmebereichs (14) in den Röntgenbildern (1, 4) führen, gewechselt wird und die Ausgabebilder (5) jeweils wenigstens aus einem Satz von Röntgenbildern (1, 4), der mit wenigstens zwei der wenigstens zwei Aufnahmeparametersätze aufgenommene Röntgenbilder (1, 4) umfasst, ermittelt werden.

## Beschreibung

Die Erfindung betrifft ein Verfahren zum Betrieb einer Röntgeneinrichtung, welche aufweist:
- eine Röntgenstrahleranordnung,
- einen Röntgendetektor zum Empfang von Röntgenstrahlung eines von der Röntgenstrahleranordnung ausgesendeten Röntgenstrahlenfeldes,
- eine Bilderzeugungskette zur Ermittlung eines Röntgenbildes eines Aufnahmebereichs aus mit dem Röntgendetektor aufgenommenen Rohdaten, und
- eine Ausgabeeinrichtung zur Ausgabe eines das Röntgenbild umfassenden oder daraus ermittelten Ausgabebildes,
wobei das Verfahren folgende Schritte umfasst:
- Aufnahme einer Abfolge von Röntgenbildern eines Aufnahmebereichs, insbesondere zur Überwachung des Aufnahmebereichs, und
- Ermittlung einer Abfolge auszugebender Ausgabebilder aus den Röntgenbildern.

Daneben betrifft die Erfindung eine Röntgeneinrichtung, ein Computerprogramm und einen elektronisch lesbaren Datenträger.

In Röntgenanwendungen ist es bekannt, über einen Zeitraum eine Abfolge von Röntgenbildern eines Aufnahmebereichs aufzunehmen, insbesondere zu Überwachungszwecken. Zur Optimierung der Darstellung können noch Bildverarbeitungsschritte durchgeführt werden, ehe das Röntgenbild oder ein daraus abgeleitetes Bild als Ausgabebild ausgebeben wird. Beispielsweise kann ein Eingriff an einem Untersuchungsobjekt und/oder eine Veränderung als Prozess überwacht werden. Bei einer solchen Überwachung können ein oder mehrere Objekte im Aufnahmebereich eine Rolle spielen. Bei Anwendungen in der Medizintechnik, insbesondere einem Patienten als Untersuchungsobjekt, werden häufig niedrige Röntgendosen eingesetzt. Eine derartige Überwachung, beispielsweise bei medizinischen Eingriffen, wird auch als fluoroskopische Überwachung beziehungsweise Fluoroskopie bezeichnet. Derart überwachte Eingriffe werden auch als bildgeführte Eingriffe bezeichnet. Beispielsweise sind in der Medizintechnik bildgeführte minimalinvasive Eingriffe bekannt.

Als Röntgeneinrichtung wird dabei häufig eine Röntgeneinrichtung mit einem C-Bogen eingesetzt, an dem sich gegenüberliegend eine Röntgenstrahleranordnung und ein Röntgendetektor angeordnet sind. Nachdem derartige C-Bogen-Röntgeneinrichtungen häufig bei Eingriffen an Gefäßen im Blutgefäßsystem zur Überwachung eingesetzt werden, sind sie auch als Angiografiesysteme bekannt.

Wesentlich bei der Überwachung von Prozessen ist meist die gute Erkennbarkeit bzw. Sichtbarkeit wenigstens eines Objekts des Untersuchungsbereichs in den Ausgabebildern. Dabei kann es sich um ein Merkmal des Untersuchungsobjekts, beispielsweise ein anatomisches Merkmal bei einem Patienten, handeln, oder aber um ein Instrument oder einen Wirkstoff, beispielsweise bei Werkstücken ein Werkzeug. Bei insbesondere minimalinvasiven Eingriffen an einem Patienten sind häufig Interventionsobjekte relevant, beispielsweise umfassend medizinische Instrumente (Nadeln, Katheter, Führungsdrähte), Implantate (Stents, Spulen, Spiralen) sowie Wirkstoffe (Kontrastmittel, Embolisierungsmittel) .

Um die Sichtbarkeit wenigstens eines relevanten Objekts sicherzustellen, ist es bekannt, Aufnahmeparameter insbesondere der Röntgenstrahleranordnung, aber auch der Bilderzeugungskette, so auszuwählen, dass die Materialeigenschaften des relevanten Objekts berücksichtigt werden und somit die Darstellung/Sichtbarkeit bei der Überwachung in den Röntgenbildern und somit Ausgabebildern verbessert, insbesondere optimiert, werden kann. Aufnahmeparameter der Röntgenstrahleranordnung umfassen beispielsweise die Röhrenspannung und den Röhrenstrom einer Röntgenröhre, die Pulsbreite, die Pulslänge bzw.

Schusszeit, zu verwendende Filterelemente für das Röntgenstrahlenfeld und dergleichen. Zusätzlich sind objektspezifische Bildverarbeitungsalgorithmen bekannt, die die Sichtbarkeit bzw. Darstellung eines Objekts verbessern können, beispielsweise, indem seine Geometrie (Form) und/oder die Geschwindigkeit, mit der es sich im Aufnahmebereich bewegt, berücksichtigt werden. Ferner können Bildverarbeitungsmaßnahmen, insbesondere in der Bilderzeugungskette verordnete Bildverarbeitungsmaßnahmen, auch auf Bewegungskorrektur und dergleichen abzielen. Beispielsweise ist bei der fluoroskopischen Überwachung eines schlagenden Herzens ein anderer zeitlicher Glättungsalgorithmus bzw. ein anders parametrierter Glättungsalgorithmus einzusetzen als bei fluoroskopischer Bildgebung des Gehirns.

Hierbei kann allerdings das Problem auftreten, dass mehrere Objekte mit unterschiedlichen Materialeigenschaften bezogen auf die Röntgenbildgebung Relevanz entfalten. Dies erschwert die Wahl geeigneter Aufnahmeparameter, um alle diese Objekte hinreichend gut darzustellen. Dabei wird diese Problematik in bekannten Lösungen häufig dem Benutzer überlassen, welcher eine geeignete Systemkonfiguration, mithin einen geeigneten Aufnahmeparametersatz, manuell wählt. In der Konsequenz führt dies zur suboptimalen Darstellung/Sichtbarkeit zumindest eines Teils der relevanten Objekte. Dies tritt auch dann ein, wenn ein auf ein relevantes Objekt einer bestimmten Objektklasse abgestimmter Aufnahmeparametersatz gewählt wird, nachdem dann andere Objekte gegebenenfalls schlecht oder sogar gar nicht sichtbar sein können.

Insbesondere sind im Bereich der Medizintechnik verschiedene Prozesse bekannt, in denen Kombinationen von Objekten in der Darstellung relevant sind, beispielsweise umfassend anatomische Merkmale und/oder Kombinationen von Interventionsobjekten. Beispielsweise werden beim sogenannten Stent-assistierten Coiling sowohl ein Stent als auch eine Spirale (Spule bzw. Coil) sowie ggf. zugehörige Instrumente überwacht. In der Schlaganfalltherapie werden beispielsweise unterschiedliche Arten von Kathetern bzw. allgemein Instrumenten eingesetzt, beispielsweise ein Aspirationskatheter und ein Stent-Retriever. Andere Arten von Instrumenten mit besonderen Materialeigenschaften, die genutzt werden, umfassen beispielsweise Ballons für Hohlraumverschlüsse und dergleichen.

Im Stand der Technik wurden bereits Lösungen vorgeschlagen, um einzelne relevante Objekte bei einer Überwachung durch Röntgenbildgebung in Ausgabebildern verbessert hervorzuheben. Beispielsweise ist unter dem Namen "ClearStent" ein Verfahren bekannt geworden, das Bilddaten der Vergangenheit zur Verbesserung der Erkennbarkeit eines Objekts in aktuellen Ausgabebildern nutzt.

Der Erfindung liegt die Aufgabe zugrunde, bei der Aufnahme einer Abfolge von Röntgenbildern eines Aufnahmebereichs mit Objekten unterschiedlicher Materialeigenschaften hinsichtlich der Röntgenbildgebung eine Verbesserung der Sichtbarkeit mehrerer solcher Objekte zu erreichen.

Diese Aufgabe wird erfindungsgemäß gelöst durch ein, insbesondere computerimplementiertes, Verfahren, eine Röntgeneinrichtung, ein Computerprogramm und einen elektronisch lesbaren Datenträger gemäß den nebengeordneten Patentansprüchen. Vorteilhafte Weiterbildungen ergeben sich aus den Unteransprüchen.

Bei einem Verfahren der eingangs genannten Art ist erfindungsgemäß vorgesehen, dass bei der Aufnahme der Röntgenbilder wiederholt zwischen wenigstens zwei Aufnahmeparametersätzen, die zur unterschiedlichen Darstellung wenigstens zweier unterschiedlicher Objekte des Aufnahmebereichs in den Röntgenbildern führen, gewechselt wird und die Ausgabebilder jeweils wenigstens aus einem Satz von Röntgenbildern, der mit wenigstens zwei der wenigstens zwei Aufnahmeparametersätze aufgenommene Röntgenbilder umfasst, ermittelt werden.

Es wird mithin vorgeschlagen, während eines Aufnahmevorgangs, also der Aufnahme der Abfolge von Röntgenbildern, mit mehreren Aufnahmeparametersätzen zu arbeiten. Insbesondere wird hierbei jeder Aufnahmeparametersatz mehrfach verwendet. Bei zwei Aufnahmeparametersätzen bedeutet dies, dass während der Aufnahme der Abfolge der Röntgenbilder nicht nur einmal von dem ersten Aufnahmeparametersatz zu dem zweiten Aufnahmeparametersatz gewechselt wird, sondern auch von dem zweiten Aufnahmeparametersatz wieder zu dem ersten Aufnahmeparametersatz zurück gewechselt wird, was wiederholt geschieht. Dies führt dazu, dass zeitlich abwechselnd unterschiedliche Aufnahmeparametersätze eingesetzt werden, aus deren Röntgenbildern in Zusammenschau Ausgabebilder ermittelt werden können, die eine gegenüber der Verwendung nur eines der Aufnahmeparametersätze verbesserte Erkennbarkeit zumindest eines der unterschiedlichen Objekte erlauben.

Hierbei wird ausgenutzt, dass im Stand der Technik inzwischen Verfahren vorgeschlagen wurden, die es erlauben, äußerst schnell zwischen Aufnahmeparametern, insbesondere auch bezüglich der Röntgenstrahleranordnung, zu wechseln. Beispielsweise kann ein Wechsel des Aufnahmeparametersatzes 60 bis 100 mal pro Sekunde möglich sein. Mithin ist es eine Kernidee der vorliegenden Erfindung, schnell zwischen unterschiedlichen Aufnahmeparametersätzen, die unterschiedliche Objekte unterschiedlich in den Röntgenbildern abbilden, zu wechseln und ein fusioniertes Ausgabebild mit erhöhter Sichtbarkeit für alle relevanten Objekte zu erzeugen. Für den Benutzer wird also ein erweitertes, verbessertes Aufnahmebild bezüglich relevanter Objekte auf der Basis der Röntgenbilder erzeugt, die mit unterschiedlichen Aufnahmeparametersätzen aufgenommen wurden.

Auf diese Weise ist es mithin möglich, auch bei unterschiedlichen Eigenschaften von Objekten bezüglich der Röntgenbildgebung eine gleichzeitige Verbesserung der Sichtbarkeit dieser mehreren Objekte zu erreichen.

Der Aufnahmebereich umfasst insbesondere wenigstens einen Teil eines Untersuchungsobjekts. Bei diesem kann es sich um ein Werkstück oder dergleichen handeln, bei dem beispielsweise Bearbeitungsprozesse oder sonstige Prozesse, beispielsweise Prüfprozesse, überwacht werden können. Besonders vorteilhaft lässt sich das Verfahren jedoch im Bereich der Medizintechnik anwenden. Dann ist das Untersuchungsobjekt insbesondere ein Patient. Die hier beschriebene Technik kann zur Überwachung physiologischer Vorgänge verwendet werden, aber auch zur Bildgebung bei, insbesondere minimalinvasiven, Eingriffen.

Hierbei lässt sich die Vorgehensweise besonders vorteilhaft auf eine fluoroskopische Überwachung (Fluoroskopie), insbesondere in der Medizintechnik, anwenden. Dabei sei dieser Stelle noch angemerkt, dass sich das vorliegende Verfahren allein mit der Bildgebung von Aufnahmebereichen durch Ansteuerung der Röntgeneinrichtung und der Verarbeitung der entstehenden Röntgenbilder befasst, mithin ein reines Bildgebungsverfahren ist, dass keinerlei diagnostische, chirurgische oder therapeutische Maßnahmen beinhaltet oder zwangsläufig erfordert.

Bei der Röntgeneinrichtung handelt es sich insbesondere um eine Röntgeneinrichtung mit einem C-Bogen, an dem sich gegenüberliegend die Röntgenstrahleranordnung und der Röntgendetektor angebracht sind. Ein C-Bogen erlaubt es, verschiedene Aufnahmegeometrien flexibel einzustellen und somit eine Projektionsrichtung zu wählen, die eine ideale Überwachung des Aufnahmebereichs erlaubt. Allgemein gesprochen können die Schritte des erfindungsgemäßen Verfahrens durch eine Steuereinrichtung der Röntgeneinrichtung durchgeführt werden, worauf im Folgenden noch näher eingegangen werden wird.

Erfindungsgemäß unterscheiden sich die Aufnahmeparametersätze bevorzugt wenigstens paarweise in wenigstens einem Aufnahmeparameter der Röntgenstrahleranordnung und/oder in wenigstens einem Aufnahmeparameter der Bilderzeugungskette. Hierbei kann der Aufnahmeparameter der Röntgenstrahleranordnung aus der Gruppe umfassend eine Röhrenspannung und/oder ein Röhrenstrom und/oder eine Pulsbreite einer Röntgenröhre der Röntgenstrahleranordnung und/oder ein zu verwendendes Filterelement der Röntgenstrahleranordnung und/oder eine Fokusgröße eines Fokuspunkts der Röntgenstrahleranordnung und/oder eine Pulslänge des Röntgenstrahlenpulses gewählt sein. Hierbei spielt die Röhrenspannung eine besonders wichtige Rolle. Vergleicht man beispielsweise ein feinmaschiges Objekt aus Eisen, beispielsweise einen Stent, mit einem gröber strukturierten Objekt aus Platin, beispielsweise einer Spirale beim Stent-Coiling, kann bei einer aufgrund eines ersten Aufnahmeparametersatzes guten Darstellung der Struktur des erstgenannten Objekts die des zweiten Objekts nicht erkennbar sein. Eine Anpassung der Röhrenspannung für einen zweiten Aufnahmeparametersatz kann eine verbesserte Erkennbarkeit der Struktur des zweiten beispielhaft genannten Objekts (ggf. so, dass man das erste Objekt nicht mehr erkennen kann) bereits ermöglichen. Eine Anpassung der Filterung der Röntgenstrahlen, beispielsweise die Wahl des Filtermaterials und/oder der Filtergeometrie, kann ebenso die Darstellbarkeit bestimmter Objekte durch Röntgenbildgebung verbessern. Auch die Fokusgröße, die Pulsbreite, die Pulslänge (bzw. bei Dauerausleuchtung die Schusszeit) und andere Parameter können im Rahmen der Erfindung variiert werden.

Jedoch erlaubt es die hier beschriebene Vorgehensweise auch, Aufnahmeparameter der Bilderzeugungskette anzupassen, um eine objektspezifische Aufbereitung des Röntgenbildes aus den Rohdaten zu ermöglichen. Hinsichtlich des Aufnahmeparameters der Bilderzeugungskette kann vorgesehen sein, dass dieser gewählt ist aus der Gruppe umfassend einen Rauschbehandlungsparameter und/oder einen Filterungsparameter und/oder einen Auflösungsparameter, insbesondere eine Ortsfrequenz, und/oder einen Korrekturparameter, insbesondere hinsichtlich einer Artefaktkorrektur und/oder einer Bewegungskorrektur. Hierbei kann insbesondere Wissen über die Objekte, die besonders gut erkennbar sein sollen, eingebracht werden. Beispielsweise kann eine Ortsfrequenz, die in der Bildverarbeitungskette verwendet wird, gemäß einer Ortsfrequenz der Strukturen von wenigstens einem der Objekte gewählt werden. Auch Informationen zur Bewegung wenigstens eines Objekts können eingehen, beispielsweise hinsichtlich einer Bewegungskorrektur/einer Bewegungsglättung. Aufnahmeparameter der Bilderzeugungskette können auch bestimmen, welche Bildverarbeitungsfunktionen angewendet werden sollen, sodass beispielsweise Bildverarbeitungsfunktionen, die bestimmte Objekte deutlicher sichtbar hervorheben, angewählt werden können.

In konkreter Ausgestaltung kann vorgesehen sein, dass die Aufnahmeparametersätze für jeweils ein Röntgenbild gemäß einer vorbestimmten Reihenfolge wechselnd genutzt werden, bei zwei Aufnahmeparametersätzen alternierend. Insbesondere wird hierbei jeder Aufnahmeparametersatz jeweils für ein Röntgenbild genutzt. Werden zwei Aufnahmeparametersätze verwendet, können mithin immer abwechselnd ein Röntgenbild mit dem ersten Aufnahmeparametersatz und ein Röntgenbild mit dem zweiten Aufnahmeparametersatz aufgenommen werden. Auch bei mehr als zwei Aufnahmeparametersätzen kann eine feste, sich wiederholende Reihenfolge angesetzt werden, beispielsweise (erster Aufnahmeparametersatz, zweiter Aufnahmeparametersatz, dritter Aufnahmeparametersatz).

In zweckmäßiger Weiterbildung kann vorgesehen sein, dass die Frequenz des Wechselns der Aufnahmeparametersätze höher gewählt ist als die Frequenz zur Ermittlung auszugebender Ausgabebilder, insbesondere derart, dass für die Ermittlung jedes Ausgabebildes genau ein Röntgenbild für jeden der Aufnahmeparametersätze aufgenommen wird. Anders gesagt kann also vorgesehen sein, dass die Aufnahmeparametersätze für die unterschiedlichen relevanten Objekte mit einer höheren zeitlichen Frequenz gewechselt werden als die angestrebte Visualisierungsfrequenz. Dies hat den Vorteil, dass der Benutzer ohne irgendwelche Einbußen hinsichtlich der Visualisierungsfrequenz eine bessere Visualisierung des Aufnahmebereichs erhält. Beispielsweise kann bei zwei Aufnahmeparametersätzen vorgesehen sein, dass die Röntgenbilder mit der doppelten Frequenz aufgenommen werden, in der die Ausgabebilder bereitgestellt werden.

Eine Variante der Erfindung kann auch vorsehen, dass die Aufnahmeparametersätze schneller gewechselt werden, als das menschliche Auge Bildwechsel auflösen kann, und dass als Ausgabebild die entstehenden Röntgenbilder mit der Frequenz ihrer Aufnahme ausgegeben werden. Auf diese Weise werden die Bildimpressionen und somit das Ausgabebild implizit durch das menschliche Gehirn zusammengesetzt.

Eine zweckmäßige Ausgestaltung der vorliegenden Erfindung sieht vor, dass zur Ermittlung des Ausgabebilds aus den Röntgenbildern unterschiedlicher Aufnahmeparametersätze
- eine, insbesondere gewichtete, wenigstens bereichsweise Überlagerung von wenigstens zwei der Röntgenbilder erfolgt und/oder
- die Röntgenbilddaten wenigstens eines Röntgenbildes in einem, insbesondere durch Segmentierung eines Objekts in demselben oder einem anderen der Röntgenbilder ermittelten, Bereich durch Röntgenbilddaten eines anderen der Röntgenbilder und/oder, insbesondere das segmentierte Objekt darstellende, Ersatzbilddaten ersetzt werden und/oder
- das Ausgabebild wenigstens teilweise pixel- und/oder patchbasiert zusammengesetzt wird.

Mithin kann das erweiterte, fusionierte Ausgabebild auf unterschiedliche Arten ermittelt werden, beispielsweise in einfach umsetzbaren Ausgestaltungen durch, insbesondere gewichtete, Mittelwertbildung über die Röntgenbilder. Denkbar ist ferner auch eine Überlagerung oder Ersetzung von Bildbereichen eines segmentierten Objekts durch ein Abbild des Objekts (Ersatzbilddaten). Ein bereichsweiser Ersatz von Röntgenbilddaten eines Röntgenbildes durch Röntgenbilddaten eines anderen Röntgenbildes oder Ersatzdaten ist insbesondere dann zweckmäßig, wenn der Darstellungsmangel eines Objekts bei Verwendung eines Aufnahmeparametersatzes darin liegt, dass dessen, insbesondere innere, Struktur nicht oder nicht hinreichend genau abgebildet wird. Ist dies jedoch in einem anderen Röntgenbild bei Verwendung eines anderen Aufnahmeparametersatzes der Fall, können dessen Röntgenbilddaten eingesetzt werden. Beispielsweise ist bei Einsatz höherer Röntgendosen zur Darstellung feinerer Strukturen der Effekt zu beobachten, dass stark schwächende Objekte statt durch ihre Struktur als ein "Schwächungsfleck" abgebildet werden, der segmentiert werden kann (hier insbesondere unter Verwendung desselben Röntgenbildes, in dem die Ersetzung vorgenommen werden soll), woraufhin Röntgenbilddaten aus einem anderen Röntgenbild eingesetzt werden können. Schließlich ist es auch möglich, dass Ausgabebild pixelweise oder Patch-weise zusammenzusetzen, insbesondere ebenso basierend auf Segmentierungsergebnissen und/oder anderen Informationen zu interessierenden Bereichen.

Im Rahmen der vorliegenden Erfindung ist es grundsätzlich denkbar, dass die wenigstens zwei Aufnahmeparametersätze zumindest für eine Überwachungsaufgabe fest vorgegeben, insbesondere objektunabhängig, sind. Anders gesagt kann also vorgesehen sein, dass wenigstens zwei festgelegte Aufnahmeparametersätze verwendet werden, unabhängig von der konkreten Bildgebungsaufgabe und den im Aufnahmebereich vorliegenden Objekten. Hierbei werden die Aufnahmeparametersätze zweckmäßigerweise so gewählt, dass sie jeweils einen breiten Bereich an Materialien, Ortsfrequenzen und dergleichen abdecken, in ihrer Kombination also möglichst viele Objekte mit akzeptabler Qualität erfassen lassen. Auf diese Weise ist eine besonders einfache Umsetzung des erfindungsgemäß vorgeschlagenen Konzeptes möglich, die dennoch eine Verbesserung gegenüber der Verwendung nur eines einzigen Aufnahmeparametersatzes ermöglicht.

Bevorzugt ist es im Rahmen der vorliegenden Erfindung jedoch, wenn jeder Aufnahmeparametersatz einer Objektklasse eines in dem Aufnahmebereich vorhandenen, ausgewählten Objekts zugeordnet, insbesondere auf diese abgestimmt, ist, wobei die Sichtbarkeit von Objekten der Objektklasse in mit dem Aufnahmeparametersatz, der der Objektklasse zugeordnet ist, aufgenommenen Röntgenbildern höher als in Röntgenbildern ist, die mit anderen Objektklassen zugeordneten Aufnahmeparametersätzen aufgenommen sind. In diesem Fall werden also im Aufnahmebereich vorliegende relevante Objekte ausgewählt, wobei die Aufnahmeparametersätze gezielt zur hochqualitativen Visualisierung von Objektklassen dieser ausgewählten Objekte ausgewählt werden. Auf diese Weise kann das Ausgabebild also zur Visualisierung der ausgewählten Objekte optimiert werden.

Dabei kann zur Beurteilung der Sichtbarkeit in einem Röntgenbild ein Sichtbarkeitsmaß, wie im Stand der Technik grundsätzlich bekannt, herangezogen werden, insbesondere um für bestimmte Objektklassen ausgewählter Objekte optimierte Aufnahmeparametersätze zu erhalten, wie dies im Stand der Technik für die Hervorhebung einzelner Objekte schon bekannt ist. Sichtbarkeitsmaße können beispielsweise den Kontrast und/oder die Erkennbarkeit der Struktur von Objekten der Objektklassen beschreiben.

Die Objektklassen können dabei letztlich beliebig konkret definiert werden. Beispielsweise ist es denkbar, die Objektklasse über bzw. als eine Objekteigenschaft, beispielsweise ein Material oder eine Materialeigenschaft, des Objekts, zu definieren. Ferner kann die Feinheit der Strukturierung des Objekts, die sichtbar gemacht werden soll, in die Definition von Objektklassen eingehen.

In dieser Ausgestaltung können, allgemein gesagt, mit anderen Worten die wenigstens zwei Aufnahmeparametersätze gezielt so gewählt werden, dass wenigstens zwei ausgewählte Objekte im Aufnahmebereich, die nicht in hinreichender Qualität, also hinreichender Sichtbarkeit, bei nur einem Aufnahmeparametersatz aufgenommen werden können, im Ausgabebild hinreichend sichtbar, insbesondere deutlich sichtbar, dargestellt werden können, insbesondere auch, was deren Struktur angeht. Die ausgewählten Objekte können einen zu überwachenden Vorgang betreffen bzw. Akteure in diesem sein. Im Beispiel der Überwachung eines minimalinvasiven Eingriffs kann ein ausgewähltes Objekt beispielsweise aus der Gruppe umfassend ein anatomisches Merkmal, ein medizinisches Instrument, ein Implantat, ein Wirkstoff und ein Hilfsmittel gewählt sein.

Die Aufnahmeparametersätze für die unterschiedlichen Objektklassen sowie, falls erforderlich, die Zuordnung von Objekten zu Objektklassen, können in wenigstens einer Datenbank gespeichert sein. Insbesondere, wie oben bereits dargelegt, können die Aufnahmeparametersätze für die Darstellung der jeweiligen wenigstens einen Objektklasse optimiert sein.

Hierbei ist es zunächst denkbar, dass die ausgewählten Objekte (und somit Objektklassen) aufgrund einer Benutzereingabe gewählt werden. Beispielsweise können bekannte Objekte in einer Benutzerschnittstelle grafisch und/oder als Text dargestellt werden, sodass ein Benutzer manuell relevante Objekte auswählen kann, die er gerne in dem Ausgabebild hinreichend deutlich sichtbar, insbesondere optimal sichtbar, sehen möchte. Die Liste bekannter Objekte kann hierbei ebenso manuell befüllt werden, bevorzugt jedoch wenigstens teilweise automatisch. Beispielsweise kann sich aus einem durchgeführten Prozess und/oder dem Untersuchungsobjekt bereits eine zugeordnete Menge an im Aufnahmebereich befindlichen Objekten ergeben. Bekannte Objekte können auch sensorisch detektiert worden sein, beispielsweise auch durch eine gezielte Kennzeichnung zur Identifikation. Beispielsweise können RFID-Chips, Strichcodes oder dergleichen von im Aufnahmebereich eingesetzten Objekten ausgelesen werden. Auch Algorithmen zur Mustererkennung können eingesetzt werden. Bei medizinischen Eingriffen an einem Patienten als Untersuchungsobjekt können aus der Art des Eingriffs bereits verwendete medizinische Instrumente, Implantate, Wirkstoffe und/oder Hilfsmittel genauso hergeleitet werden wie anatomische Merkmale. Fehlende Informationen, beispielsweise Materialien, Modelle und dergleichen sowie fehlende Objekte, können beispielsweise, wie beschrieben, sensorisch und/oder durch Benutzereingabe ergänzt werden.

In besonders vorteilhafter Weiterbildung der vorliegenden Erfindung kann jedoch vorgesehen sein, dass die Objekte wenigstens teilweise automatisch aufgrund einer Priorisierung bekannter Objekte, bei der allen bekannten Objekten ein Prioritätswert zugeordnet wird, ausgewählt werden. Die Ermittlung der bekannten Objekte kann dabei wie oben bereits zur benutzerseitigen Auswahl von bekannten Objekten aus Listen beschrieben erfolgen. Zweckmäßigerweise werden hierbei Objekte, die einer bezüglich den Aufnahmeparametersätzen gleichen Objektklasse zugeordnet sind, als ein gemeinsames bekanntes Objekt betrachtet, so dass eine doppelte Wahl derselben Objektklasse vermieden wird. Dies kann auch so formuliert werden, dass die Priorisierung für Objektklassen anhand des größten Prioritätswerts der ihr zugehörigen Objekte (der dann das ausgewählte Objekt definiert) erfolgt.

Eine Priorisierung kann derart erfolgen, dass allen bekannten Objekten ein Prioritätswert zugeordnet wird. Es wird eine Anzahl der am höchsten priorisierten der bekannten Objekte verschiedener Objektklasse automatisch ausgewählt. Die Priorisierung kann fest vorgegeben sein oder über die Zeit veränderlich, worauf im Folgenden noch näher eingegangen werden wird. Die Zahl der auszuwählenden Objekte und somit Objektklassen kann über einen zu überwachenden Prozess konstant bzw. fest vorgegeben sein. Beispielsweise kann eine Anzahl, beispielsweise zwei, auszuwählender Objekte vorgegeben sein, so dass diese Anzahl höchst priorisierter Objekte ausgewählt wird. Die Anzahl kann aber auch variieren, beispielsweise wenn ein Schwellwert der Priorität zusätzlich oder alternativ verwendet wird. Es sind auch (zusätzliche) Zeitintervalle denkbar, in denen nur ein ausgewähltes Objekt existiert, also auch nur ein Aufnahmeparametersatz verwendet wird.

Dabei sei an dieser Stelle noch angemerkt, dass die automatische Auswahl wenigstens eines Objekts zur besonders gut sichtbaren Darstellung bei der Röntgenüberwachung auch unabhängig von dem Wechsel von Aufnahmeparametersätzen vorteilhaft sein kann. Mit anderen Worten ist ein Verfahren zum Betrieb einer Röntgeneinrichtung denkbar, wobei die Röntgeneinrichtung aufweist:
- eine Röntgenstrahleranordnung,
- einen Röntgendetektor zum Empfang von Röntgenstrahlung eines von der Röntgenstrahleranordnung ausgesendeten Röntgenstrahlenfeldes,
- eine Bilderzeugungskette zur Ermittlung eines Röntgenbildes eines Aufnahmebereichs aus mit dem Röntgendetektor aufgenommenen Rohdaten, und
- eine Ausgabeeinrichtung zur Ausgabe eines das Röntgenbild umfassenden oder daraus ermittelten Ausgabebildes, wobei das Verfahren folgende Schritte umfasst:
- Aufnahme einer Abfolge von Röntgenbildern eines Aufnahmebereichs, insbesondere zur Überwachung des Aufnahmebereichs, und
- Ermittlung einer Abfolge auszugebender Ausgabebilder aus den Röntgenbildern,
dadurch gekennzeichnet, dass das Verfahren ferner aufweist: -
- Auswahl wenigstens eines Objekts aufgrund einer Priorisierung bekannter, in dem Aufnahmebereich vorhandener Objekte, bei der allen bekannten Objekten ein Prioritätswert zugeordnet wird,
- für jedes ausgewählte Objekt, Auswahl wenigstens eines einer Objektklasse des ausgewählten Objekts zugeordneten, insbesondere auf diese abgestimmten, Aufnahmeparametersatzes, wobei die Sichtbarkeit von Objekten der Objektklasse in mit dem Aufnahmeparametersatz, der der Objektklasse zugeordnet ist, aufgenommenen Röntgenbildern höher als in Röntgenbildern ist, die mit anderen Objektklassen zugeordneten Aufnahmeparametersätzen aufgenommen sind, und
- Aufnahme der Röntgenbilder unter Verwendung des wenigstens einen ausgewählten Aufnahmeparametersatzes.

Dabei lassen sich selbstverständlich alle vorangehenden und folgenden Ausführungen auch auf ein solches denkbares Verfahren anwenden. Mit einem solchen denkbaren Verfahren sind insbesondere auch Ausgestaltungen möglich, die sich immer nur auf ein ausgewähltes Objekt beziehen und darauf abzielen, immer eine hervorragende Darstellung dieses relevantesten Objekts zu erzielen.

Mehrere relevante, mithin ausgewählte, Objekte können bei dem denkbaren Verfahren auch über eine Aufteilung in Zeitabschnitte, in denen das als Ausgabebild verwendete Röntgenbild mit jeweils einem der zugeordneten Aufnahmeparametersätze aufgenommen wird, verbessert dargestellt werden. Sind die Zeitabschnitte gleich lang, kann von einem "regulär time slicing" gesprochen werden. Möglich ist aber auch ein "irregulär time slicing", bei dem die Länge der Zeitabschnitte beispielsweise gemäß der Prioritätswerte gewählt werden kann. So wird ein hochrelevantes ausgewähltes Objekt in einem relativ großen Anteil der Röntgenbilder optimiert dargestellt, während das wenigstens eine ausgewählte Objekt, das weniger Aufmerksamkeit benötigt, seltener optimiert dargestellt wird.

Eine automatische Priorisierung und somit Auswahl von Objekten, auf deren Sichtbarkeit hin das Ausgabebild optimiert werden soll, entlastet den Benutzer von einer Tätigkeit und erlaubt auch eine dynamische Anpassung, wenn im Verlauf beispielsweise eines überwachten Prozesses andere Objekte größere Relevanz entfalten als bisher dargestellte Objekte. Besonders vorteilhaft ist eine Ausgestaltung mit automatischer Auswahl von Objekten (und somit Aufnahmeparametersätzen) dann, wenn der Benutzer an dem Prozess beteiligt ist, beispielsweise für den Fall eines Eingriffes am Untersuchungsobjekt. Dann muss der Benutzer seine Konzentration nicht unterbrechen und erhält dennoch durch Betrachten des Ausgabebildes immer die Informationen, die er auch tatsächlich benötigt. Insbesondere können bei einem Eingriff beispielsweise die Objekte ausgewählt werden, die gerade bewegt werden und/oder mit denen gerade interagiert wird. Insbesondere bei der im Folgenden noch genauer diskutierten Aktualisierung der Auswahl, mithin dynamisch veränderlicher Auswahl von Objekten, können jedoch auch gezielt solche bekannten Objekte ausgewählt werden, die der Aufmerksamkeit des Benutzers bedürfen.

Allgemein sei angemerkt, dass grundsätzlich, also unabhängig von den bei der Überwachung eines jeweiligen Prozesses eingesetzten Aufnahmeparametersätzen, Objekten bzw. Objektklassen, die bei allen Aufnahmeparametersätzen hinreichend sichtbar sind, ein niedrigerer Prioritätswert zugeordnet werden kann als Objekten, die bei Verwendung mancher Aufnahmeparametersätze schlechter oder gar nicht sichtbar sind. So wird die Sichtbarkeit schlechter sichtbarer Objekte verbessert/sichergestellt. Ist beispielsweise ein Objekt einfacher, ausgedehnter Struktur aus Metall im Aufnahmebereich vorhanden, ist davon auszugehen, dass dieses auch unabhängig vom Aufnahmeparametersatz hinreichend gut sichtbar ist, sodass eine hohe Priorisierung hier nicht notwendig ist. Es sind auch Ausführungsbeispiele denkbar, in denen für die Priorisierung bekannte Objekte faktisch ausgeschlossen werden können, beispielsweise deren Prioritätswert grundsätzlich auf die niedrigste mögliche Priorität gesetzt werden kann. Dies gilt neben ohnehin hinreichend deutlich sichtbaren Objekten insbesondere auch für bekannte Objekte, für die aufgrund der Art des zu überwachenden Prozesses bekannt ist, dass ihre Darstellung nicht relevant ist. Dies gilt beispielsweise bei minimalinvasiven medizinischen Eingriffen für anatomische Merkmale, die nicht betroffen sind, und/oder anatomische Merkmale, die ohnehin, beispielsweise aus einem Vorabbilddatensatz, als Overlay dem Ausgabebild hinzugefügt werden.

Ein Ausschluss von bekannten Objekten bzw. eine Minimal-Priorisierung für bekannte Objekte kann auch auf Basis einer Augen-Nachverfolgung (Eye-Tracking) eines Benutzers erfolgen. Beispielsweise können mithin als auswertbare Objekte nur solche herangezogen werden, welche sich in einem Bereich befinden, den der Benutzer betrachtet. So wird der Benutzer dort unterstützt, war gerade hinsieht, in dem die relevanten Objekte in diesem Bereich besonders gut erkennbar dargestellt werden.

Eine Weiterbildung der vorliegenden Erfindung kann vorsehen, dass die Ermittlung des Prioritätswerts der Priorisierung für wenigstens ein bekanntes Objekt unter Berücksichtigung einer Dynamikinformation, die eine aktuelle Dynamik des Objekts beschreibt, erfolgt. Dabei kann eine solche Dynamikinformation jede Art von denkbaren Veränderungen betreffen, beispielsweise Starrkörperbewegungen (Rotationen, Translationen) genauso wie Formänderungen, Ausdehnung und dergleichen. Der Berücksichtigung einer Dynamikinformation liegt der Gedanke zugrunde, dass eine hohe Dynamik aufweisende bekannte Objekte in dem Prozess üblicherweise auch eine relevante Rolle spielen, wie beispielsweise für bewegte Instrumente, sich verbreitende und/oder verbrauchende Wirkstoffe, durch externen Einfluss bewegte Merkmale und dergleichen gilt. Mit anderen Worten gilt für viele Prozesse, dass eine hohe Dynamik auch eine hohe Relevanz anzeigt. Während für die Dynamikinformation verschiedenste Quellen denkbar sind, insbesondere Nachverfolgungs-Sensorik, Positionsbestimmungssysteme und dergleichen, ist es erfindungsgemäß doch bevorzugt, die Dynamikinformation wenigstens teilweise aus Röntgenbildern zu ermitteln.

So kann vorgesehen sein, dass zur Ermittlung der Dynamikinformation wenigstens ein das jeweilige bekannte Objekt zeigendes Röntgenbild, insbesondere eine Zeitserie von Röntgenbildern, durch eine, insbesondere trainierte, Dynamikermittlungsfunktion ausgewertet wird. Auf diese Weise kann beispielsweise ein Instrument, das ein Benutzer gerade bewegt, identifiziert werden oder aber ein gerade aktiver Wirkstoff erkannt werden. Dabei sind die Bewegung von Objekten ermittelnde bzw. Objekte nachverfolgende Dynamikermittlungsfunktionen im Stand der Technik bereits grundsätzlich bekannt, beispielsweise bei der Nachverfolgung von Objekten im Straßenverkehr und dergleichen. Entsprechende Dynamikermittlungsfunktionen können auch im Rahmen der vorliegenden Erfindung eingesetzt werden, um automatisiert auf einfache Art und Weise die Dynamikinformation zu ermitteln.

Die Dynamikermittlungsfunktion kann mittels Maschinenlernen trainiert sein. Im Allgemeinen bildet eine trainierte Funktion kognitive Funktionen ab, die Menschen mit anderen menschlichen Gehirnen assoziieren. Durch Training basierend auf Trainingsdaten (Maschinenlernen) ist die trainierte Funktion in der Lage, sich an neue Umstände anzupassen und Muster zu detektieren und zu extrapolieren.

Allgemein gesagt können Parameter einer trainierten Funktion durch Training angepasst werden. Insbesondere können überwachtes Lernen, halbüberwachtes Lernen, nicht überwachtes Lernen, Reinforcement Learning und/oder aktives Lernen verwendet werden. Darüber hinaus kann auch Repräsentationslernen (auch als "feature learning" bekannt) eingesetzt werden. Die Parameter der trainierten Funktion können insbesondere iterativ durch mehrere Trainingsschritte angepasst werden.

Eine trainierte Funktion kann beispielsweise ein neuronales Netz, eine Support Vector Machine (SVM), einen Entscheidungsbaum und/oder ein Bayes-Netzwerk umfassen und/oder die trainierte Funktion kann auf k-means-Clustering, Q-Learning, genetischen Algorithmen und/oder Zuordnungsregeln basieren. Insbesondere kann ein neuronales Netzwerk ein tiefes neuronales Netzwerk, ein Convolutional Neural Network (CNN) oder ein tiefes CNN sein. Darüber hinaus kann das neuronale Netzwerk ein Adversarial Network, ein tiefes Adversarial Network und/oder ein Generative Adversarial Network (GAN) sein.

Im vorliegenden Fall der Bildverarbeitung bietet sich für die Dynamikermittlungsfunktion insbesondere ein CNN an.

Insbesondere bei einem Einsatz des Verfahrens im medizintechnischen Bereich kann der Aufnahmebereich selbst zumindest teilweise einer grundsätzlich vorhandenen Bewegung, mithin beispielsweise einer physiologischen Bewegung wie der Atmung und/oder dem Herzschlag, unterworfen sein. In solchen Fällen kann zweckmäßigerweise vorgesehen sein, dass bei einem einer physiologischen Bewegung unterworfenen Aufnahmebereich diese bei Ermittlung der Dynamikinformation berücksichtigt, insbesondere herausgerechnet wird. Hierzu kann eine Bewegungskorrektur und/oder eine Bewegungsseparation erfolgen.

Verschiedene konkrete Ansätze zur Ermittlung der Dynamikinformation durch die Dynamikermittlungsfunktion können im Rahmen dieser Ausgestaltung eingesetzt werden. So kann in einer ersten Ausführungsform beispielsweise vorgesehen werden, künstliche Intelligenz, beispielsweise also einen trainierten Anteil der Dynamikermittlungsfunktion, zur Erkennung von (bekannten) Objekten in den Röntgenbildern der Zeitserie einzusetzen. Basierend hierauf können dann bekannte Methoden der Bewegungsdetektion bzw. Objektnachverfolgung eingesetzt werden, beispielsweise Methoden des optischen Flusses. Denkbar ist jedoch auch eine Ausführungsform, in der die trainierte Dynamikermittlungsfunktion als Eingangsdaten die Röntgenbilder der Zeitserie, gegebenenfalls annotiert, erhält, sodass sie als Ausgabedaten Bewegungsfelder (insbesondere objektspezifisch) und/oder Heat Maps liefern kann. Möglich ist ferner auch eine Ausführungsform, in der eine trainierte Dynamikermittlungsfunktion allein auf Basis der Zeitserie von Röntgenbildern bereits die Objekten zugeordnete Dynamikinformation als Ausgangsdaten liefert.

In konkreter Ausgestaltung kann als Dynamikinformation wenigstens eine Geschwindigkeit und/oder ein Geschwindigkeitsverlauf des Objekts ermittelt werden. Geschwindigkeiten können beispielsweise in Pixelabständen zwischen einzelnen Röntgenbildern pro Zeiteinheit ermittelt werden. Insbesondere bei der Ermittlung von Geschwindigkeiten und/oder Geschwindigkeitsverläufen ist es zweckmäßig, physiologische Bewegungen zu separieren und/oder zu korrigieren. Hierzu sei allgemein angemerkt, dass gerade im Hinblick auf eine Bewegungsseparation künstliche Intelligenz auch vorteilhaft eingesetzt werden kann, um beispielsweise Herzbewegung und/oder Atembewegung von sonstigen, insbesondere den konkreten Prozess betreffenden Bewegungen zu separieren.

Bei der Ermittlung der Dynamikinformation für ein fluidisches Objekt, beispielsweise einen Embolisationsmittelbolus oder einen Kontrastmittelbolus, kann eine Ausbreitungsfront des fluidischen Objekts nachverfolgt werden und/oder ein Gradient der Konzentration des fluidischen Objekts ermittelt werden. Auch für fluidische Objekte, für die starre Bewegungen keine oder wenig Rolle spielen, ist es mithin möglich, sinnvolle Dynamikinformationen zu bestimmen, die Hinweise auf deren Relevanz geben. Beispielsweise existiert nach der Verabreichung eines Wirkstoffs, wie beispielsweise einem Kontrastmittel oder einem Embolisationsmittel, zunächst eine Phase hoher Dynamik mit signifikanten Änderungen, die entsprechend über die Bewegung der Bolus-Front und/oder Konzentrationsänderungen erfasst werden kann.

Ausgestaltungen der vorliegenden Erfindung können ferner vorsehen, dass die Ermittlung des Prioritätswerts der Priorisierung für wenigstens ein bekanntes Objekt unter Berücksichtigung einer einen überwachten Prozess betreffenden Workflowinformation erfolgt. Hierbei kann insbesondere vorgesehen sein, dass die Workflowinformation durch eine trainierte Workflowermittlungsfunktion ermittelt wird. Es kann mithin eine auf künstlicher Intelligenz basierende Detektion eines aktuellen Workflow-Schrittes erfolgen, wie dies im Stand der Technik allgemein bereits vorgeschlagen worden ist, beispielsweise zur Bereitstellung kontextsensitiver Hilfe. Eine derartige Ermittlung der Workflowinformation nutzt beispielsweise Eingangsdaten verschiedener Sensoren der Prozessumgebung, insbesondere Eingriffsumgebung, und auch die Röntgenbildgebung selbst, um festzustellen, wo in einem den Prozess beschreibenden Workflow man sich gerade befindet. Hieraus wiederum kann gefolgert werden, welche Objekte gerade Relevanz besitzen und welche Objekte für die aktuelle Situation weniger relevant sind. Insbesondere können basierend auf der Workflowinformation auch einige Objekte, insbesondere Interventionsobjekte, von der Menge der bekannten Objekte ausgeschlossen werden bzw. ihr Prioritätswert die niedrigste Priorität anzeigend gewählt werden. Betrifft ein aktueller Workflow-Schritt beispielsweise die Platzierung eines Implantats oder Hilfsmittels im Aufnahmebereich, während sich ein Instrument mit einem anderen Hilfsmittel in einer Warteposition im Aufnahmebereich befindet, können das erstgenannte Implantat oder Hilfsmittel sowie dessen Instrument höher priorisiert werden als das andere Instrument mit dem anderen Hilfsmittel.

Dabei sei im Hinblick auf die zu wechselnden Aufnahmeparametersätze an dieser Stelle noch angemerkt, dass es durchaus denkbar ist, die Prioritätswerte auch in den Wechselvorgang selbst eingehen zu lassen, beispielsweise derart, dass eine Gewichtung der Nutzung der Aufnahmeparametersätze gemäß den Prioritätswerten erfolgt. Ist beispielsweise ein erstes ausgewähltes Objekt doppelt so hoch priorisiert wie ein zweites ausgewähltes Objekt, kann der dem ersten ausgewählten Objekt (bzw. dessen Objektklasse) zugeordnete Aufnahmeparametersatz doppelt so häufig verwendet werden wie der dem zweiten ausgewählten Objekt (bzw. dessen Objektklasse) zugeordnete Aufnahmeparametersatz. Dies ist jedoch hauptsächlich bei stark unterschiedlichen Prioritätswerten sinnvoll.

In besonders zweckmäßige Ausgestaltung bei automatischer Auswahl der Objekte/Objektklassen ist eine dynamische Aktualisierung vorgesehen. Das bedeutet, es kann vorgesehen sein, dass eine Aktualisierung der bekannten Objekte und/oder Priorisierungswerte sowie der ausgewählten Objekte in regelmäßigen Zeitabständen, insbesondere nach jedem n-ten Röntgenbild und/oder Ausgabebild, erfolgt, oder dass eine Aktualisierung ereignisbasiert erfolgt.

In einer ersten Variante ist hierbei vorgesehen, dass eine periodische Aktualisierung erfolgt. Beispielsweise kann nach der Aufnahme jedes n-ten Röntgenbildes oder Ausgabebildes eine Aktualisierung erfolgen; bevorzugt nach jeder Ermittlung eines Ausgabebildes. Somit kann immer zeitaktuell auf eine Änderung von Prioritäten reagiert werden.

Möglich ist es aber auch, sozusagen adaptiv eine Umschaltung bei Eintritt eines Ereignisses zu überprüfen. Konkret kann hierbei vorgesehen sein, dass als Ereignis das Eintreten eines benutzerdefinierten Triggers verwendet wird. In diesem Fall kann beispielsweise mittels einer Benutzerschnittstelle ein Benutzer einen oder mehrere Trigger definieren, bei deren Eintritt überprüft wird, ob eine Aktualisierung der ausgewählten Objekte notwendig ist. Ferner kann vorgesehen sein, dass das Eintreten des Ereignisses unter Verwendung der Dynamikinformation und/oder der oder einer weiteren Workflowinformation ermittelt wird. So kann beispielsweise bei einem Wechsel der Workflow-Situation, beispielsweise bei einem Wechsel zu einem anderen Workflow-Schritt, überprüft werden, ob eine Aktualisierung der ausgewählten Objekte notwendig ist. Zudem gibt beispielsweise die Dynamikinformation ja grundsätzlich an, welche Objekte besonders aktiv sind. Liegt hier ein Wechsel vor, wird also ein anderes bekanntes Objekt aktiv, während ein bisher aktives Objekt in seiner Aktivität nachlässt, kann ebenso eine Aktualisierung getriggert werden.

Ferner kann vorgesehen sein, dass als Ereignis das Vorliegen eines extern bereitgestellten Triggersignals verwendet wird. So kann beispielsweise ein Injektor, beispielsweise für ein Kontrastmittel, ein Triggersignal an die Röntgeneinrichtung senden, dass ein Kontrastmittel gegeben wurde, worauf hin das Kontrastmittel höher priorisiert werden kann und die Aufnahmeparameter so gewählt werden können, dass das Kontrastmittel im Ausgabebild so gut wie möglich dargestellt wird. In einem anderen Beispiel kann bei Verwendung einer Robotereinrichtung, beispielsweise endovaskulär oder perkutan, diese die Röntgeneinrichtung über das konkrete Interventionsobjekt, das bewegt wird, informieren. Dieses kann dann auch höher priorisiert werden.

Eine allgemeine Weiterbildung der vorliegenden Erfindung kann vorsehen, dass zur Erhöhung der Sichtbarkeit wenigstens eines bekannten, insbesondere ausgewählten, hervorzuhebenden Objekts unter Verwendung einer Bildinformation des Objekts in vergangenen Röntgenbildern eine Objekterscheinungsinformation, insbesondere statistisch, ermittelt wird und ein aktuelles, insbesondere mit dem dem hervorzuhebenden Objekt zugeordneten Aufnahmeparametersatz aufgenommenes, Röntgenbild und/oder das aktuelle Ausgabebild unter Verwendung der Objekterscheinungsinformation modifiziert wird, insbesondere durch Einsetzen der Objekterscheinungsinformation in einem Bildbereich des hervorzuhebenden Objekts. Eine solche Vorgehensweise ist insbesondere dann zweckmäßig, wenn das Objekt grundsätzlich schwer zu erkennen ist, beispielsweise verrauscht ist oder dergleichen. Auf diese Weise wird eine verbesserte, deutlich sichtbare Darstellung des Objekts unter Hinzunahme vergangener Bildinformationen des Objekts ermittelt und im Ausgabebild verwendet. So kann beispielsweise bei einem verrauschten Objekt eine deutlich verbesserte Kontrastierung erreicht werden. Hierbei kann insbesondere vorgesehen sein, dass die Objekterscheinungsinformation auch unter Verwendung der Bildinformation des Objekts in dem aktuellen Röntgenbild ermittelt wird, also so viel Information wie möglich einbezogen wird.

Konkret kann beispielsweise vorgesehen sein, dass die Bildinformationen der verschiedenen Röntgenbilder zur Ermittlung der Objekterscheinungsinformation gemittelt werden. Anders gesagt findet also eine Verbesserung der Sichtbarkeit des Objekts basierend auf zeitlicher Mittelung der Bildinformation statt, um Objektdetails zu extrahieren und hervorzuhebenden und (statistisches) Rauschen zu reduzieren.

Bei einer, beispielsweise physiologischen, Bewegung im Aufnahmebereich kann es zur Ermittlung der Objekterscheinungsinformation notwendig sein, eine Registrierung der Röntgenbilder zueinander, insbesondere basierend auf wenigstens einem Merkmal des Objekts, vorzunehmen. Mithin können, insbesondere bei einer Bewegung des hervorzuhebenden Objekts, die vergangenen Röntgenbilder und, falls verwendet, das aktuelle Röntgenbild miteinander registriert werden.

Neben dem Verfahren betrifft die Erfindung auch eine Röntgeneinrichtung, aufweisend:
- eine Röntgenstrahleranordnung,
- einen Röntgendetektor zum Empfang von Röntgenstrahlung eines von der Röntgenstrahleranordnung ausgesendeten Röntgenstrahlenfeldes,
- eine Bilderzeugungskette zur Ermittlung eines Röntgenbildes eines Aufnahmebereichs aus mit dem Röntgendetektor aufgenommenen Rohdaten,
- eine Ausgabeeinrichtung zur Ausgabe eines das Röntgenbild umfassenden oder daraus ermittelten Ausgabebildes, und
- eine Steuereinrichtung
wobei die Steuereinrichtung umfasst:
- eine Aufnahmeeinheit zur Aufnahme einer Abfolge von Röntgenbildern eines Aufnahmebereichs, insbesondere zur Überwachung des Aufnahmebereichs, und
- eine Ermittlungseinheit zur Ermittlung einer Abfolge auszugebender Ausgabebilder aus den Röntgenbildern.

Dabei ist die Aufnahmeeinheit bei der Aufnahme der Röntgenbilder zum wiederholten Wechseln zwischen wenigstens zwei Aufnahmeparametersätzen, die zur unterschiedlichen Darstellung wenigstens zweier unterschiedlicher Objekte des Aufnahmebereichs in den Röntgenbildern führen, und die Ermittlungseinheit zur Ermittlung der Ausgabebilder jeweils wenigstens aus einem Satz von Röntgenbildern, der mit wenigstens zwei der wenigstens zwei Aufnahmeparametersätze aufgenommene Röntgenbilder umfasst, ausgebildet.

Die Steuereinrichtung weist wenigstens einen Prozessor und wenigstens ein Speichermittel auf. Sie umfasst Funktionseinheiten, die durch Hardware und/oder Software gebildet sein können und insbesondere auch Schritte des erfindungsgemäßen Verfahrens ausführen können. Die Aufnahmeeinheit steuert dabei gemäß der Aufnahmeparametersätze die Röntgenstrahleranordnung, den Röntgendetektor und die Bilderzeugungskette zur Aufnahme von Röntgenbildern an. Die Ermittlungseinheit umfasst geeignete Bildverarbeitungsmittel, um Ausgabebilder zu erzeugen. Weitere Funktionseinheiten zur Durchführung weiterer, optionaler Schritte des erfindungsgemäßen Verfahrens sind ebenso denkbar. Insbesondere kann eine Auswahleinheit zur Auswahl der Objekte, insbesondere auf Basis der Priorisierung, vorgesehen sein. Die Auswahleinheit gibt über die ausgewählten Objekte bzw. deren Objektklassen der Aufnahmeeinheit die zu verwendenden Aufnahmeparametersätze vor.

Bei der Röntgeneinrichtung kann es sich, wie bereits dargelegt, insbesondere und bevorzugt um eine C-Bogen-Röntgeneinrichtung, insbesondere als Teil einer Angiografieumgebung, handeln.

Ein erfindungsgemäßes Computerprogramm ist direkt in ein Speichermittel einer Steuereinrichtung einer Röntgeneinrichtung ladbar und weist Programmmittel derart auf, dass bei Ausführung des Computerprogramms auf der Steuereinrichtung diese veranlasst wird, die Schritte eines erfindungsgemäßen Verfahrens durchzuführen. Das Computerprogramm kann auf einem elektronisch lesbaren Datenträger gemäß der Erfindung gespeichert sein, welcher mithin darauf gespeicherte Steuerinformationen umfasst, die wenigstens ein erfindungsgemäßes Computerprogramm umfassen und derart ausgestaltet sind, dass bei Verwendung des Datenträgers in einer Steuereinrichtung einer Röntgeneinrichtung diese ausgebildet wird, ein erfindungsgemäßes Verfahren durchzuführen. Bei dem Datenträger handelt es sich insbesondere um einen nichttransienten Datenträger, beispielsweise eine CD-ROM.

Weitere Vorteile und Einzelheiten der vorliegenden Erfindung ergeben sich aus den im Folgenden beschriebenen Ausführungsbeispielen sowie anhand der Zeichnung. Dabei zeigen:
- Fig. 1: einen Ablaufplan eines Ausführungsbeispiels des erfindungsgemäßen Verfahrens,
- Fig. 2: schematisch ein erstes, mit einem ersten Aufnahmeparametersatz aufgenommenes Röntgenbild,
- Fig. 3: schematisch ein zweites, mit einem zweiten Aufnahmeparametersatz aufgenommenes Röntgenbild,
- Fig. 4: schematisch ein aus dem ersten und dem zweiten Röntgenbild ermitteltes Ausgabebild,
- Fig. 5: eine Prinzipskizze einer erfindungsgemäßen Röntgeneinrichtung, und
- Fig. 6: den funktionalen Aufbau einer Steuereinrichtung der Röntgeneinrichtung.

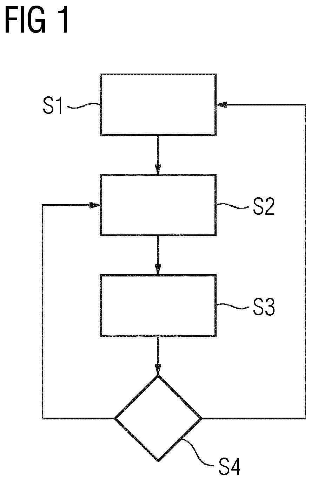

Fig. 1 zeigt einen Ablaufplan eines Ausführungsbeispiels des erfindungsgemäßen Verfahrens. Dabei wird vorliegend zur beispielhaften Erläuterung die Bildüberwachung eines minimalinvasiven, Stent-assistierten Coiling-Eingriffes im Bereich eines Gehirnaneurysmas diskutiert, ohne dass dies die allgemeine Anwendbarkeit des Verfahrens infrage stellen soll.

Bei dem in Fig. 1 dargestellten Ausführungsbeispiel wird ein Prozess in einem Aufnahmebereich durch Röntgenbildgebung überwacht. Dabei sollen relevante Objekte für einen Benutzer optimiert sichtbar dargestellt werden. Hierzu werden zunächst in einem Schritt S1 die Objekte bzw. Objektklassen aus einer Liste von bekannten Objekten ausgewählt, für die die Röntgenbildgebung optimiert stattfinden soll. Hierzu wird vorliegend eine Priorisierung verwendet.

Die Liste von bekannten Objekten im Aufnahmebereich bzw. vorkommenden Objektklassen wird beispielsweise in einer Auswahleinheit einer Steuereinrichtung der verwendeten Röntgeneinrichtung verwaltet. Die Anwesenheit von Objekten im Aufnahmebereich und somit die Liste von bekannten Objekten kann aus einer Vielzahl von Informationen hergeleitet werden. So gibt beispielsweise bereits der Prozess selbst, der überwacht werden soll, deutliche Hinweise, welche Objekte im Aufnahmebereich beispielsweise des Untersuchungsobjekts bereits vorhanden sind und welche Objekte, im Rahmen eines Eingriffs beispielsweise Interventionsobjekte, zusätzlich für den Prozess dort vorliegen. Im Rahmen eines bestimmten Workflows, dem gefolgt wird, kann dies noch weiter präzisiert werden.

Beispielsweise kann mittels einer insbesondere trainierten Workflowermittlungsfunktion eine Workflowinformation bestimmt werden, die beispielsweise einen aktuellen Workflow-Schritt anzeigen kann. Diesem wiederum können hierfür im Aufnahmebereich benötigte Objekte zugeordnet sein. Die Workflowinformation kann auch hinsichtlich der Bestimmung von Prioritätswerten für die Priorisierung verwendet werden.

Die Anwesenheit von Objekten im Aufnahmebereich kann auch aus einer Benutzereingabe und/oder sensorisch ermittelt werden. Auch aus Röntgenbildern kann auf im Aufnahmebereich anwesende Objekte gefolgert werden, beispielsweise durch Detektion der Objekte in den Röntgenbildern durch geeignete, insbesondere trainierte, Detektionsfunktionen, wie sie im Stand der Technik grundsätzlich bekannt sind.

In einem Speichermittel der Steuereinrichtung der Röntgeneinrichtung ist dabei auch eine Datenbank abgelegt, die optional Objekten Objektklassen zuordnet, in jedem Fall aber Objektklassen Aufnahmeparametersätze zuordnet, die auf Objekte der Objektklasse abgestimmte Aufnahmeparameter für eine Röntgenstrahleranordnung und eine Bilderzeugungskette der Röntgeneinrichtung umfassen. Das bedeutet, die Aufnahmeparameter sind unter Berücksichtigung der für die Röntgenbildgebung relevanten Eigenschaften der Objekte der Objektklasse, beispielsweise deren Material und deren Struktur (beispielsweise auftretende Ortsfrequenzen), so gewählt, dass die Objekte der Objektklasse mit dem dieser zugeordneten Aufnahmeparametersatz besser sichtbar dargestellt werden als mit anderen Objektklassen zugeordneten Aufnahmeparametersätzen. Insbesondere sind die Aufnahmeparameter zur Optimierung der Sichtbarkeit der Objekte der Objektklasse gewählt.

Treten nun unter den bekannten Objekten mehrere Objekte auf, die derselben einer solchen Objektklasse zuzuordnen sind, werden diese als ein bekanntes Objekt gehandhabt; mit anderen Worten werden letztlich die Objektklassen priorisiert und ausgewählt. Hierbei wird der höchste vorhandene Prioritätswert der bekannten Objekte der Objektklasse für die Objektklasse bzw. das zusammengefasste Gesamtobjekt verwendet, wobei alle bekannten Objekte derselben Objektklasse also als ein einziges auswählbares bekanntes Objekt mit dem maximalen Prioritätswert der einzelnen bekannten Objekte zählen. So wird jede Objektklasse nur einmal gewählt, in dem nur deren repräsentativstes Objekt, also das mit der höchsten Priorität, auswählbar ist. Es kann jedoch auch der Auswahlprozess so gestaltet werden, dass bei mehreren auszuwählenden Objekten diese nicht derselben Objektklasse angehören können.

Bestimmten Objekten kann dabei auch grundsätzlich der niedrigste mögliche Prioritätswert zugeordnet werden, oder sie können der Betrachtung sogar völlig entzogen werden. Dies kann beispielsweise für Objekte gelten, die anderweitig in das Ausgabebild aufgenommen werden (beispielsweise anatomische Merkmale, die aus einem Vorabbilddatensatz überlagert werden), aber auch für solche, für die beispielsweise aus der Workflowinformation bekannt ist, dass sie nicht für die aktuelle Workflow-Situation relevant sind. Auch ist es im Übrigen denkbar, den Blick des Benutzers nachzuverfolgen (Eye-Tracking) und nur Objekte im Blickbereich des Benutzers mit einem das Minimum überschreitenden Prioritätswert zu versehen.

Allgemein wird zur Ermittlung der Prioritätswerte für bekannte Objekte zumindest die bereits erwähnte Workflowinformation und eine Dynamikinformation zu dem jeweiligen bekannten Objekt verwendet. Die Dynamikinformation beschreibt die Dynamik des Objekts, wobei bei hoher Dynamik von einer höheren Relevanz ausgegangen wird. Auch die Workflowinformation beschreibt, welche bekannten Objekte in der aktuellen Workflow-Situation wie relevant sind. Die Dynamikinformation wird dabei vorliegend aus einer Zeitserie von Röntgenbildern, die das entsprechende Objekt zeigen, ermittelt, wobei die Zeitserie von Röntgenbildern, insbesondere bereits annotiert, als Eingangsdaten einer, insbesondere trainierten, Dynamikermittlungsfunktion verwendet werden. Bei physiologischen Bewegungen werden diese insbesondere korrigiert oder im Rahmen einer Bewegungsseparation separiert. Die Dynamikinformation kann, insbesondere bei festen Objekten, eine Geschwindigkeit oder einen Geschwindigkeitsverlauf, umfassen. Bei fluidischen Objekten, beispielsweise einem Bolus, kann die Bewegung einer Bolus-Front und/oder eine Veränderung der Konzentration als Dynamikinformation ermittelt werden. Wird beispielsweise im genannten Beispiel des Stent-assistierten Coiling das medizinische Instrument, insbesondere der Katheter, mit der Coil bewegt, spricht dies dafür, dass die Coil platziert werden soll und somit diese und gegebenenfalls der entsprechende Katheter relevante Objekte darstellen. Der Prioritätswert wird entsprechend hoch gewählt. Auch weitere Informationen können in die Bestimmung der Prioritätswerte eingehen, beispielsweise wie oben beschrieben, um auszuschließende bekannte Objekte zu definieren.

Im Schritt S1 können dann die bekannten Objekte im Aufnahmebereich ausgewählt werden, die den höchsten Prioritätswert, also höchste Priorität, aufweisen (und unterschiedlichen Objektklassen angehören). Dabei kann eine feste Anzahl auszuwählender Objekte, beispielsweise zwei, vorgesehen sein, jedoch können auch variierende Anzahlen, beispielsweise bei Schwellwerten für den Prioritätswert zur Auswahl, resultieren.

In einem Schritt S2 werden dann Röntgenbilder aufgenommen, und zwar unter Verwendung von den Aufnahmeparametersätzen der Datenbank, die den ausgewählten Objekten/Objektklassen zugeordnet sind. Hierbei wird im vorliegenden Ausführungsbeispiel in einer vorgegebenen Reihenfolge jeweils genau ein Röntgenbild mit jedem Aufnahmeparametersatz durch entsprechende Ansteuerung der Röntgenstrahleranordnung, des Röntgendetektors und der Bilderzeugungskette aufgenommen. Beispielsweise werden bei zwei ausgewählten Objekten immer abwechselnd Röntgenbilder mit den Aufnahmeparametersätzen aufgenommen.

Es wird im Schritt S2 also mit hoher Geschwindigkeit zwischen Aufnahmeparametersätzen gewechselt. Im vorliegenden Ausführungsbeispiel umfassen die Aufnahmeparameter der Aufnahmeparametersätze sowohl zwischen Aufnahmeparametersätzen paarweise unterschiedliche Aufnahmeparameter für die Röntgenstrahleranordnung als auch paarweise unterschiedliche Aufnahmeparameter für die Bilderzeugungskette. Aufnahmeparameter, die für unterschiedliche Objektklassen unterschiedlich gewählt werden, können für die Röntgenstrahleranordnung eine Röhrenspannung und/oder ein Röhrenstrom einer Röntgenröhre der Röntgenstrahleranordnung und/oder ein zu verwendendes Filterelement der Röntgenstrahleranordnung und/oder eine Fokusgröße eines Fokuspunkts der Röntgenstrahleranordnung umfassen, für die Bilderzeugungskette einen Rauschbehandlungsparameter und/oder einen Filterungsparameter und/oder einen Auflösungsparameter und/oder einen Korrekturparameter, insbesondere hinsichtlich einer Artefaktkorrektur und/oder einer Bewegungskorrektur. Zwischen den Aufnahmen hierbei mit einer Frequenz gewechselt, die höher ist als eine Frequenz, in der im Schritt S3 Ausgabebilder ermittelt und ausgegeben werden. Beispielsweise können Röntgenbilder mit einer Rate von 60 bis 100 fps aufgenommen werden, insbesondere 80 fps, wobei für jedes Röntgenbild (jeden Frame) zwischen den vorliegend zwei Aufnahmeparametersätzen gewechselt wird. Hierbei werden im Stand der Technik für andere Zwecke grundsätzlich bekannte Techniken der schnellen Umschaltung, insbesondere hinsichtlich der Röntgenstrahleranordnung, genutzt.

Grundsätzlich sind sogar Ausgestaltungen denkbar, in denen so schnell zwischen Aufnahmeparametersätzen gewechselt wird, dass bei unmittelbarer Ausgabe der Röntgenbilder an einer Ausgabeeinrichtung die menschlich wahrnehmbare Änderungsfrequenz überschritten wird und hierdurch im Geist des Betrachters implizit das Ausgabebild durch Kombination der Röntgenbilder verschiedener Aufnahmeparametersätze entsteht.

Im vorliegenden Fall allerdings existiert in der Steuereinrichtung eine Ermittlungseinheit, die einen Satz von Röntgenbildern, der für jeden Aufnahmeparametersatz wenigstens ein, vorliegend genau ein, Röntgenbild, das mit diesem Aufnahmeparametersatz aufgenommen wurde, umfasst, zu einem Ausgabebild, in dem alle ausgewählten Objekte deutlich zu erkennen sind, in einem Schritt S3 verarbeitet und dieses ausgibt.

Es wird im Schritt S3 mithin aus den Objektklassen-Röntgenbildern, die als für ein ausgewähltes Objekt spezifische Röntgenbilder aufgefasst werden können, ein für den Benutzer hinsichtlich aller ausgewählter Objekte verbessertes Ausgabebild erzeugt. Dies kann beispielsweise durch, insbesondere gewichtete, pixelweise Mittelwertbildung der einzelnen Röntgenbilder, Überlagerung segmentierter ausgewählter Objekte auf eines der Röntgenbilder, Patch-basierte Erzeugung basierend auf interessierenden Bereichen und/oder pixelbasierte Erzeugung geschehen. Insbesondere kann vorgesehen sein, bereichsweise Röntgenbilddaten eines der Röntgenbilder durch Röntgenbilddaten eines anderen der Röntgenbilder zu ersetzen.

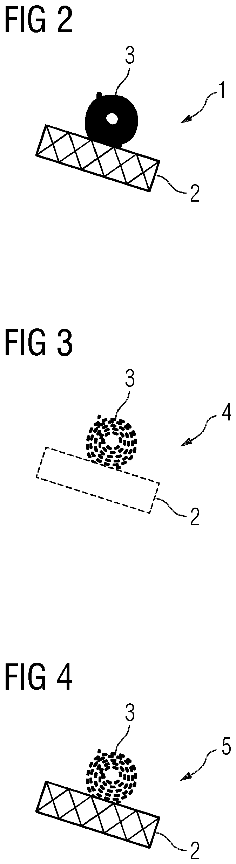

Dies ist beispielhaft für einen speziellen Anwendungsfall in den Figuren 2 bis 4 dargestellt. Hierbei zeigt Fig. 2 schematisch ein erstes Röntgenbild 1, dass mit einem ersten, für einen Stent 2 als ausgewähltes Objekt bzw. Objektklasse optimierten Aufnahmeparametersatz aufgenommen wurde. Ersichtlich ist der vorliegend aus Eisen bzw. Stahl bestehende Stent deutlich, auch hinsichtlich seiner feinmaschigen Struktur, zu erkennen. Im Aufnahmebereich befindet sich jedoch als weiteres ausgewähltes Objekt auch eine Spirale 3 (Coil), die aus deutlich stärker schwächendem Platin besteht und unter Verwendung des ersten Aufnahmeparametersatzes ohne Auflösung der Struktur letztlich "fleckartig" erscheint.

Fig. 3 zeigt ein zweites Röntgenbild 4, dass mit einem zweiten, auf die Spirale 3 abgestimmten Aufnahmeparametersatz aufgenommen wurde. Ersichtlich ist in diesem Röntgenbild 4 der Stent 2 kaum zu erkennen, allerdings wird die Struktur der Spirale 3 korrekt aufgelöst.

Fig. 4 zeigt das aus den Röntgenbildern 1 und 4 ermittelte Ausgabebild 5, in dem sowohl der Stent 2 mit seiner Struktur als auch die Spirale 3 mit ihrer Struktur deutlich sichtbar und erkennbar sind. Beispielsweise kann das Ausgabebild 5 erzeugt werden, indem die Spirale 3 im Röntgenbild 1 segmentiert wird und durch die Röntgenbilddaten der Spirale 3 aus dem Röntgenbild 4 ersetzt wird. Jedoch sind, wie oben beschrieben, auch andere Ansätze zur Generierung des Ausgabebilds 5 im Schritt S3 möglich.

Dabei sei an dieser Stelle noch angemerkt, dass bei der Erzeugung des Ausgabebilds für manche oder alle ausgewählten Objekte, insbesondere solche, die grundsätzlich schwer in der Röntgenbildgebung gut darstellbar sind, beispielsweise verrauscht sind, eine Verbesserung dadurch erzielt werden kann, dass für ein solches hervorzuhebendes Objekt eine Bildinformation des Objekts in vergangenen Röntgenbildern verwendet wird, um eine Objekterscheinungsinformation, insbesondere durch statistische Mittelwertbildung unter Verwendung auch des aktuellen Röntgenbilds, zu ermitteln und ein aktuelles, insbesondere mit dem dem hervorzuhebenden Objekt zugeordneten Aufnahmeparametersatz aufgenommenes, Röntgenbild 1, 4 und/oder das aktuelle Ausgabebild 5 unter Verwendung der Objekterscheinungsinformation zu modifizieren. Hierbei kann insbesondere die Objekterscheinungsinformation in einem Bildbereich des hervorzuhebenden Objekts statt der aktuellen Bildinformation eingesetzt werden. So wird eine objektspezifische Verbesserung der Darstellung/Sichtbarkeit basierend auf zeitliche Mittelung der Bildinformation erreicht, indem Objektdetails extrahiert und verstärkt werden und statistisches Rauschen reduziert wird. Diese Verbesserung kann auch für mehrere ausgewählte Objekte, insbesondere simultan, erzielt werden.

In einem Schritt S4 wird überprüft, ob eine Aktualisierungsbedingung erfüllt ist. Dabei kann zum einen vorgesehen sein, dass eine Aktualisierung der bekannten Objekte, der Priorisierungswerte sowie der ausgewählten Objekte in regelmäßigen Zeitabständen erfolgen, beispielsweise nach jedem n-ten Röntgenbild 1, 4 oder jedem n-ten Ausgabebild 5. Denkbar ist jedoch auch, in der Aktualisierungsbedingung zusätzlich oder alternativ zu überprüfen, ob ein bestimmtes Ereignis eingetreten ist, mithin eine ereignisbasiert Aktualisierung vorzusehen. Als Ereignis kann das Eintreten eines benutzerdefinierten Triggers verwendet werden.

Besonders bevorzugt und im vorliegenden Ausführungsbeispiel vorgesehen ist es jedoch, wenn das Eintreten des Ereignisses unter Verwendung der Dynamikinformation und der Workflowinformation ermittelt wird. Tritt bei einem anderen Objekt beispielsweise die höchste Dynamik auf, spricht dies dafür, dass dieses Objekt nun relevanter ist. Genauso kann der Eintritt in einen neuen Workflow-Schritt bedeuten, dass sich die Relevanz von Objekten verändert. In allen solchen Fällen ist es zweckmäßig, die Liste bekannter Objekte und ihrer Prioritätswert zu aktualisieren und entsprechend die ausgewählten Objekte zu überprüfen und gegebenenfalls zu verändern.

Denkbar ist es ferner auch, als Ereignis das Vorliegen eines externen Triggersignals, beispielsweise von einem Wirkstoff-Injektor und/oder einer Robotikeinrichtung, zu überprüfen. Es sei angemerkt, dass derartige externe Informationen insbesondere auch in die Ermittlung der Workflowinformation und/oder der Dynamikinformation eingehen können.

Ist die Aktualisierungsbedingung erfüllt, wird mit Schritt S 1 fortgefahren, ist sie nicht erfüllt, wird der Aufnahmeprozess fortgesetzt. Es sei angemerkt, dass im Schritt S4 selbstverständlich auch eine Abbruchbedingung überprüft werden kann, bei deren Eintreten die Bildaufnahme und somit die Überwachung des Prozesses beendet wird. Die Abbruchbedingung kann eine Benutzereingabe auswerten, aber auch automatisch überwacht werden, beispielsweise wiederum anhand der Workflowinformation.

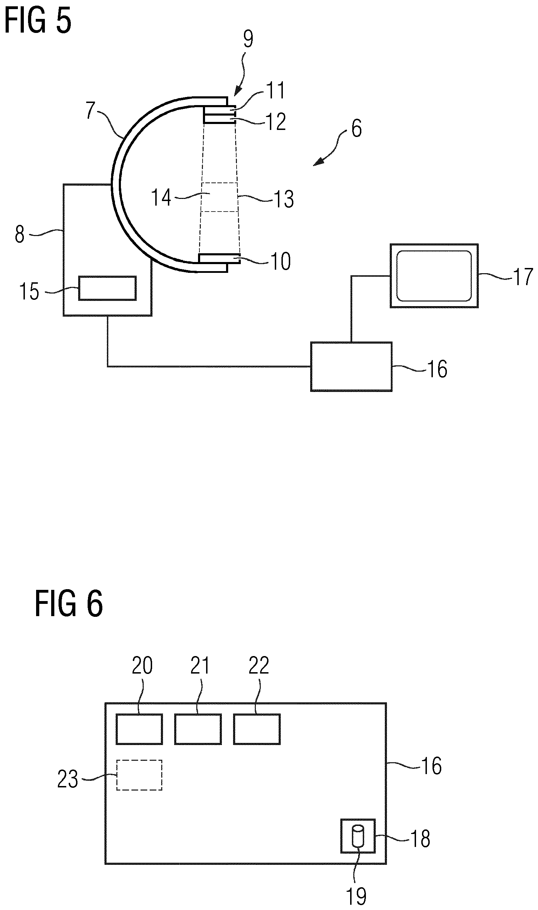

Fig. 5 zeigt eine Prinzipskizze einer erfindungsgemäßen Röntgeneinrichtung 6. Die Röntgeneinrichtung 6 umfasst einen C-Bogen 7, der an einem Stativ 8 bewegbar gehaltert sein kann. An dem C-Bogen 7 sind sich gegenüberliegend eine Röntgenstrahleranordnung 9 und ein Röntgendetektor 10 angeordnet. Die Röntgenstrahleranordnung 9 umfasst vorliegend eine Röntgenröhre 11 und eine Filteranordnung 12 mit verschiedenen, wechselbaren Filterelementen. Mittels der Röntgenstrahleranordnung 9 kann ein Röntgenstrahlenfeld 13 erzeugt werden, dessen Röntgenstrahlen von dem Röntgendetektor 10 empfangen und vermessen werden. Ein Untersuchungsobjekt, beispielsweise ein Patient, kann beispielsweise mittels eines hier nicht näher gezeigten Patiententisches derart im Röntgenstrahlenfeld 13 positioniert werden, dass der Aufnahmebereich 14 erfasst werden kann. In dem Aufnahmebereich 14 befindliche Objekte sind der Übersichtlichkeit halber nicht näher gezeigt.

Mit dem Röntgendetektor 10 erfasste Rohdaten werden mittels einer Bilderzeugungskette 15 verarbeitet, um Röntgenbilder 1, 4 zu überzeugen.

Der Betrieb der Röntgeneinrichtung 6 wird durch eine Steuereinrichtung 16 gesteuert, die insbesondere auch zur Durchführung des erfindungsgemäßen Verfahrens ausgebildet ist. Die Steuereinrichtung 16 ist ferner mit einer Ausgabeeinrichtung 17, beispielsweise einem Monitor, verbunden, auf dem Röntgenbilder 1, 4 bzw. Ausgabebilder 5 angezeigt werden können.

Die Röntgeneinrichtung 6 kann insbesondere Teil eines medizinischen Eingriffs-Arbeitsplatzes sein, beispielsweise einer Angiografieumgebung. Bei Patienten als Untersuchungsobjekt erfolgt die Überwachung durch die Röntgenbilder insbesondere fluoroskopisch. Die weitere, hier nicht mehr dargestellte Komponenten aufweisen, beispielsweise Sensorik, eine Injektor, eine Robotikeinrichtung und dergleichen.

Fig. 6 zeigt den funktionalen Aufbau der Steuereinrichtung 16 genauer. Diese umfasst zunächst ein Speichermittel 18, in dem beispielsweise die Datenbank 19 mit den Objektklassen zugeordneten Aufnahmeparametersätzen gespeichert sein kann. Auch sonstige Informationen, insbesondere Bilddaten, können dort abgelegt werden.

Mittels einer Auswahleinheit 20 wird, wie zu Schritt S1 beschrieben, die Liste bekannter Objekte mit den Prioritätswerten verwaltet und es werden Objekte ausgewählt, die deutlich sichtbar, insbesondere optimiert, dargestellt werden sollen. In einer Aufnahmeeinheit 21 erfolgt dann die Aufnahme von Röntgenbildern 1, 4 gemäß Schritt S2 durch Ansteuerung der Röntgenstrahleranordnung 9, des Röntgendetektors 10 und der Bilderzeugungskette 15. In einer Ermittlungseinheit 22 kann, insbesondere bei zwei ausgewählten Objekten aus je einem Paar von Röntgenbildern 1, 4, gemäß Schritt S3 eine Ermittlung und Ausgabe von Ausgabebilder 5 erfolgen.

Eine übergeordnete Steuerung der Umsetzung des hier beschriebenen Verfahrens kann durch die Auswahleinheit 20 erfolgen, es kann jedoch auch optional eine geordnete Steuereinheit 23 vorgesehen sein, die dann insbesondere auch die Aktualisierungsbedingung sowie die Abbruchbedingung gemäß Schritt S4 überwachen kann.

Es können auch weitere, hier nicht genauer dargestellte Funktionseinheiten vorgesehen sein, beispielsweise weitere Ermittlungseinheiten zur Ermittlung der Workflowinformation und der Dynamikinformation und/oder die Steuereinrichtung 16 kann wenigstens eine Schnittstelle zum Empfang externer Trigger, insbesondere hinsichtlich der Aktualisierungsbedingung und/oder der Abbruchbedingung, umfassen.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

Unabhängig vom grammatikalischen Geschlecht eines bestimmten Begriffes sind Personen mit männlicher, weiblicher oder anderer Geschlechteridentität mit umfasst.

## Patentansprüche

1. Verfahren zum Betrieb einer Röntgeneinrichtung (6), welche aufweist:
- eine Röntgenstrahleranordnung (9),
- einen Röntgendetektor (10) zum Empfang von Röntgenstrahlung eines von der Röntgenstrahleranordnung (9) ausgesendeten Röntgenstrahlenfeldes (13),
- eine Bilderzeugungskette (15) zur Ermittlung eines Röntgenbildes (1, 4) eines Aufnahmebereichs (14) aus mit dem Röntgendetektor (10) aufgenommenen Rohdaten, und
- eine Ausgabeeinrichtung (17) zur Ausgabe eines das Röntgenbild (1, 4) umfassenden oder daraus ermittelten Ausgabebildes (5),
wobei das Verfahren folgende Schritte umfasst:
- Aufnahme einer Abfolge von Röntgenbildern (1, 4) eines Aufnahmebereichs (14), insbesondere zur Überwachung des Aufnahmebereichs (14), und
- Ermittlung einer Abfolge auszugebender Ausgabebilder (5) aus den Röntgenbildern (1, 4),
**dadurch gekennzeichnet, dass** bei der Aufnahme der Röntgenbilder (1, 4) wiederholt zwischen wenigstens zwei Aufnahmeparametersätzen, die zur unterschiedlichen Darstellung wenigstens zweier unterschiedlicher Objekte des Aufnahmebereichs (14) in den Röntgenbildern (1, 4) führen, gewechselt wird und die Ausgabebilder (5) jeweils wenigstens aus einem Satz von Röntgenbildern (1, 4), der mit wenigstens zwei der wenigstens zwei Aufnahmeparametersätze aufgenommene Röntgenbilder (1, 4) umfasst, ermittelt werden.

2. Verfahren nach Anspruch 1, **dadurch gekennzeichnet, dass** sich die Aufnahmeparametersätze paarweise in wenigstens einem Aufnahmeparameter der Röntgenstrahleranordnung (9) und/oder in wenigstens einem Aufnahmeparameter der Bilderzeugungskette (15) unterscheiden.

3. Verfahren nach Anspruch 2, **dadurch gekennzeichnet, dass** der Aufnahmeparameter der Röntgenstrahleranordnung (9) gewählt ist aus der Gruppe umfassend eine Röhrenspannung und/oder ein Röhrenstrom und/oder eine Pulsbreite einer Röntgenröhre (11) der Röntgenstrahleranordnung (9) und/oder ein zu verwendendes Filterelement der Röntgenstrahleranordnung (9) und/oder eine Fokusgröße eines Fokuspunkts der Röntgenstrahleranordnung (9) und/oder eine Pulslänge des Röntgenstrahlenpulses und/oder der Aufnahmeparameter der Bilderzeugungskette (15) gewählt ist aus der Gruppe umfassend einen Rauschbehandlungsparameter und/oder einen Filterungsparameter und/oder einen Auflösungsparameter und/oder einen Korrekturparameter, insbesondere hinsichtlich einer Artefaktkorrektur und/oder einer Bewegungskorrektur.

4. Verfahren nach einem der vorangehenden Ansprüche, **dadurch gekennzeichnet, dass** die Aufnahmeparametersätze für jeweils ein Röntgenbild (1, 4) gemäß einer vorbestimmten Reihenfolge wechselnd genutzt werden, bei zwei Aufnahmeparametersätzen alternierend.

5. Verfahren nach Anspruch 4, **dadurch gekennzeichnet, dass** die Frequenz des Wechselns der Aufnahmeparametersätze höher gewählt ist als die Frequenz zur Ermittlung auszugebender Ausgabebilder (5), insbesondere derart, dass für die Ermittlung jedes Ausgabebildes (5) genau ein Röntgenbild (1, 4) für jeden der Aufnahmeparametersätze aufgenommen wird.

6. Verfahren nach einem der vorangehenden Ansprüche, **dadurch gekennzeichnet, dass** zur Ermittlung des Ausgabebilds (5) aus den Röntgenbildern (1, 4) unterschiedlicher Aufnahmeparametersätze
- eine, insbesondere gewichtete, wenigstens bereichsweise Überlagerung von wenigstens zwei der Röntgenbilder (1, 4) erfolgt und/oder
- die Röntgenbilddaten wenigstens eines Röntgenbildes (1, 4) in einem, insbesondere durch Segmentierung eines Objekts in demselben oder einem anderen der Röntgenbilder (1, 4) ermittelten, Bereich durch Röntgenbilddaten eines anderen der Röntgenbilder (1, 4) und/oder, insbesondere das segmentierte Objekt darstellende, Ersatzbilddaten ersetzt werden und/oder
- das Ausgabebbild (5) wenigstens teilweise pixel- und/oder patchbasiert zusammengesetzt wird.

7. Verfahren nach einem der vorangehenden Ansprüche, **dadurch gekennzeichnet, dass** jeder Aufnahmeparametersatz einer Objektklasse eines in dem Aufnahmebereich vorhandenen, ausgewählten Objekts zugeordnet, insbesondere auf diese abgestimmt, ist, wobei die Sichtbarkeit von Objekten der Objektklasse in mit dem Aufnahmeparametersatz, der der Objektklasse zugeordnet ist, aufgenommenen Röntgenbildern (1, 4) höher als in Röntgenbildern (1, 4) ist, die mit anderen Objektklassen zugeordneten Aufnahmeparametersätzen aufgenommen sind.

8. Verfahren nach Anspruch 7, **dadurch gekennzeichnet, dass** die ausgewählten Objekte wenigstens teilweise automatisch aufgrund einer Priorisierung bekannter Objekte, bei der allen bekannten Objekten ein Prioritätswert zugeordnet wird, ausgewählt werden.

9. Verfahren nach Anspruch 8, **dadurch gekennzeichnet, dass** die Ermittlung des Prioritätswerts der Priorisierung für wenigstens ein bekanntes Objekt unter Berücksichtigung einer Dynamikinformation, die eine aktuelle Dynamik des Objekts beschreibt, erfolgt.

10. Verfahren nach Anspruch 9, **dadurch gekennzeichnet, dass** zur Ermittlung der Dynamikinformation wenigstens ein das jeweilige bekannte Objekt zeigendes Röntgenbild (1, 4), insbesondere eine Zeitserie von Röntgenbildern (1, 4), durch eine, insbesondere trainierte, Dynamikermittlungsfunktion ausgewertet wird.

11. Verfahren nach einem der Ansprüche 8 bis 10, **dadurch gekennzeichnet, dass** die Ermittlung des Prioritätswerts der Priorisierung für wenigstens ein bekanntes Objekt unter Berücksichtigung einer einen überwachten Prozess betreffenden Workflowinformation erfolgt.

12. Verfahren nach einem der Ansprüche 8 bis 11, **dadurch gekennzeichnet, dass** eine Aktualisierung der bekannten Objekte und/oder Priorisierungswerte sowie der ausgewählten Objekte in regelmäßigen Zeitabständen, insbesondere nach jedem n-ten Röntgenbild (1, 4) und/oder Ausgabebild (5), erfolgt, oder dass eine Aktualisierung ereignisbasiert erfolgt.

13. Röntgeneinrichtung (6), aufweisend:
- eine Röntgenstrahleranordnung (9),
- einen Röntgendetektor (10) zum Empfang von Röntgenstrahlung eines von der Röntgenstrahleranordnung (9) ausgesendeten Röntgenstrahlenfeldes (13),
- eine Bilderzeugungskette (15) zur Ermittlung eines Röntgenbildes (1, 4) eines Aufnahmebereichs (14) aus mit dem Röntgendetektor (10) aufgenommenen Rohdaten,
- eine Ausgabeeinrichtung (17) zur Ausgabe eines das Röntgenbild (1, 4) umfassenden oder daraus ermittelten Ausgabebildes (5), und
- eine Steuereinrichtung (16)
wobei die Steuereinrichtung (16) umfasst:
- eine Aufnahmeeinheit (21) zur Aufnahme einer Abfolge von Röntgenbildern (1, 4) eines Aufnahmebereichs (14), insbesondere zur Überwachung des Aufnahmebereichs (14), und
- eine Ermittlungseinheit (22) zur Ermittlung einer Abfolge auszugebender Ausgabebilder (5) aus den Röntgenbildern (1, 4),
**dadurch gekennzeichnet, dass** die Aufnahmeeinheit (21) bei der Aufnahme der Röntgenbilder (1, 4) zum wiederholten Wechseln zwischen wenigstens zwei Aufnahmeparametersätzen, die zur unterschiedlichen Darstellung wenigstens zweier unterschiedlicher Objekte des Aufnahmebereichs (14) in den Röntgenbildern (1, 4) führen, und die Ermittlungseinheit (22) zur Ermittlung der Ausgabebilder (5) jeweils wenigstens aus einem Satz von Röntgenbildern (1, 4), der mit wenigstens zwei der wenigstens zwei Aufnahmeparametersätze aufgenommene Röntgenbilder (1, 4) umfasst, ausgebildet ist.

14. Computerprogramm, welches Programmmittel derart aufweist, dass bei Ausführung des Computerprogramms auf einer Steuereinrichtung (16) einer Röntgeneinrichtung (6) diese veranlasst wird, die Schritte eines Verfahrens nach einem der Ansprüche 1 bis 12 durchzuführen.

15. Elektronisch lesbarer Datenträger, auf dem ein Computerprogramm nach Anspruch 14 gespeichert ist.
